# EUROPEAN PATENT SPECIFICATION

(11) **EP 4 103 332 B1**
(45) Date of publication and mention of the grant of the patent: **02.07.2025**
(21) Application number: 20811418.1
(22) Date of filing: 27.11.2020
(51) Int. Cl.: B05B 1/00, B05B 1/14, B05B 11/00

(54) **AEROSOL ASSEMBLY FOR A MEDICAMENT DELIVERY DEVICE**
AEROSOLANORDNUNG FÜR EINE MEDIKAMENTENABGABEVORRICHTUNG
ENSEMBLE D'AÉROSOL POUR DISPOSITIF D'ADMINISTRATION DE MÉDICAMENTS

(30) Priority: 13.02.2020 EP 20157044
(43) Date of publication of application: 21.12.2022
(73) Proprietor: SHL Medical AG, 6302 Zug (CH)
(72) Inventor: SÄLL, Daniel, 131 28 Nacka Strand (SE)
(86) International application number: PCT/EP2020/083738
(87) International publication number: WO 2021/160308

(56) References cited:
- EP-A1- 2 275 160
- WO-A1-03/097139
- WO-A1-2012/007315
- WO-A1-97/12683

## Description

### TECHNICAL FIELD

The present disclosure generally relates to aerosol assemblies for medicament delivery devices.

### BACKGROUND

Medicament delivery devices may be provided with a micro nozzle to create an aerosol of the therein contained medicament for example for inhalation. The nozzle is subjected to high pressure during medicament administration. US7837235 B2 discloses a device for clamping a fluidic component. The fluidic component is arranged in an elastomeric shaped part, the contour of which is matched to the outer contour of the component and to the inner contour of a holder. The elastomeric part is chamfered towards the fluidic component on its pressure side. When the holder is assembled the elastomeric shaped part is deformed by a projection provided on the mating part and is put under uniformly distributed internal tension, after the elastomeric shaped parts surrounds the fluidic component to its full height. This "floating mounting" means that there are no unacceptable local tension peaks and no deformation of the component. The mounting is sealed against the fluid even when the fluid pressure fluctuates repeatedly from a very low level to several 100 bar. Further known aerosol assemblies are disclosed in WO 2012/007315 A1 and EP 2 275 160 A1.

### SUMMARY

It is in general desirable to get rid of all air in a pressurised medicament delivery device during priming as trapped air will cause "drooling" on top of the nozzle after dosing. The drooling may create a crust on the nozzle when it dries, which may cause clogging.

An object of the present disclosure is to provide an aerosol assembly which solves, or at least mitigates problems of the prior art.

There is hence according to a first aspect of the present disclosure provided an aerosol assembly for a medicament delivery device, the aerosol assembly comprising: a nozzle chip assembly including a carrier and a nozzle chip provided on the carrier, a holding structure having a fluid channel extending through the holding structure, wherein the nozzle chip assembly is configured to be mounted into the fluid channel such that fluid flowing through the fluid channel passes through the nozzle chip, and an elastic sealing member configured to be arranged with a tight fit in the fluid channel, the sealing member having a central through-opening configured to receive the nozzle chip to seal the nozzle chip towards the carrier.

The elastic sealing member seals the nozzle chip towards the carrier, whereby the amount of air trapped around the nozzle chip is minimised. "Drooling" may thereby be reduced, and thus the risk of crust building up on the nozzle chip causing clogging is also reduced.

The nozzle chip is preferably a micro nozzle.

The nozzle chip may for example be glued onto the carrier.

The sealing member may bear against the inner surface of the fluid channel to obtain the tight fit with the fluid channel. The sealing member may hence be in direct contact with the inner surface of the fluid channel.

According to the invention the fluid channel has an inner surface provided with a circumferentially extending opening, wherein the carrier has an outer boundary portion mounted into the opening to hold the carrier in place. The carrier and hence the nozzle chip can thereby be held in place axially relative to the holding structure.

The opening may for example be in the form of a circumferentially extending slit configured to receive the outer boundary portion of the carrier. The carrier may be a planar substrate.

Luer lock interfaces are commonly used in medicament delivery devices. One drawback with Luer locks is that the thread is not self-locking. It is intended to be easily unscrewed to change e.g. a needle tip on a syringe. It has been found that there is a high risk that the Luer lock will come loose and leak after storage. The applicant has made tests that involved storage of a medicament delivery device with a Luer lock connection at an elevated temperature of 55° C for 48 hours. It was found that the Luer lock threads unscrewed themselves during this time.

Another issue is that at high pressures, there is a risk that the Luer threads unscrew due to the force from the pressure. The applicant carried out a test with a male Luer lock member made of polypropylene (PP) and female Luer lock member made of PP and one made of polyethylene (PE). The female PP Luer lock member started to leak/come loose at 50 bar and the female PE Luer lock member started to leak/come loose at 20 bar. One way to overcome this problem is to alter the pitch. However, such modification is not always feasible as for example the threads will be small and difficult to mould/assemble with high yield.

On the positive side, an advantage with Luer lock connections is that they are easy to manufacture and give a strong and self-aligning interface. From this perspective, it is hence advantageous to use Luer lock connections. It would however be desirable to use Luer lock connections without the aforementioned drawbacks.

Therefore, the invention comprises a base member having a Luer cone configured to extend into the fluid channel towards the nozzle chip assembly, the Luer cone having a central channel extending through the base member and configured to extend coaxially with the fluid channel, wherein the base member and the holding structure have Luer threads by which the base member and the holding structure are configured to be connected, wherein the central through-opening of the sealing member is configured to open towards the central channel, and wherein the sealing member is configured to be arranged axially between the Luer cone and the carrier.

Thus, instead of using the Luer cone for sealing, the nozzle chip is sealed by means of the elastic sealing member. At the same time, the Luer connection provides a strong and self-aligning interface.

According to one embodiment the sealing member is configured to be in contact with the Luer cone and with the carrier. This contact may be a direct contact.

According to one embodiment the sealing member comprises a viscoelastic material.

According to one embodiment the viscoelastic material is an elastomer.

According to one embodiment the sealing member is rotationally symmetric.

According to one embodiment the central through-opening has a constant inner diameter along its entire extension.

The sealing member may have a first end surface which faces the carrier, wherein the first end surface is provided with a first groove that extends in the circumferential direction around the central through-opening of the sealing member. The first groove may hence function as a liquid collector in which dissipating liquid may accumulate, preventing any liquid that has started to dissipate in between the carrier and the first end surface to dissipate further.

The first end surface may bear against the carrier.

The sealing member may have a second end surface arranged at an opposite end of the sealing member relative to the first end surface, wherein the second end surface is provided with a second groove that extends in the circumferential direction around the central through-opening of the sealing member.

The second end surface may bear against an end face of the Luer cone. The second groove may hence function as a liquid collector in which dissipating liquid may accumulate, preventing any liquid that has started to dissipate in between the second end surface and the end face of the Luer cone to dissipate further.

According to one embodiment the sealing member is configured to be mounted in the fluid channel with a mounting compression of between 0.2 to 0.6 mm. This means that the sealing member is compressed between 0.2 and 0.6 mm when arranged in the fluid channel. In particular, the outer surface of the sealing member is compressed in this manner. The tight fit with the inner surface of the fluid channel is thereby achieved.

According to one embodiment the sealing member has an external surface provided with a rib extending in a circumferential direction of the sealing member. The rib is elastic and deformed when the sealing member is arranged in the fluid channel. The rib further reduces the risk of liquid flow between the sealing member and the inner surface of the fluid channel. According to one embodiment the holding structure has a hollow generally cylindrical end portion in which the fluid channel extends axially, wherein the cylindrical end portion has an external surface provided with first Luer threads, and wherein the base member has an outer wall arranged concentrically with the Luer cone, wherein the cylindrical end portion is configured to be arranged concentrically between the Luer cone and the outer wall, the outer wall having second Luer threads configured to engage with the first Luer threads.

As an alternative to the aforementioned Luer lock solution, not being covered by the subject-matter of the claims, the base member and the holding structure may be configured to interlock with each other by means of a snap-fit mechanism.

By means of the sealing member the snap-fit mechanism will be able to withstand high pressure and the holding member will therefore not disengage from the base member, which would otherwise be a risk.

The base member may be provided with a plurality of gripping arms and the holding structure may be provided with radial protrusions, wherein each gripping arm is configured to engage with a respective radial protrusion, the gripping arms and the radial protrusions forming the snap-fit mechanism.

The gripping arms may be configured to extend parallel with the central longitudinal axis of the aerosol assembly.

The gripping arms may be configured to flex radially.

There is according to a second aspect of the present disclosure provided a medicament delivery device comprising an aerosol assembly according to the first aspect.

According to one embodiment the medicament delivery device is an inhaler.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 shows an example of a medicament delivery device comprising an aerosol assembly;
Fig. 2 a longitudinal section of the medicament delivery device in Fig. 1;
Fig. 3 shows a perspective view of an example of a mouthpiece attached to an aerosol assembly;
Fig. 4 shows a perspective view of the mouthpiece and the aerosol assembly in Fig. 3;
Fig. 5 is a top view of the aerosol assembly in Fig. 3;
Fig. 6 is a longitudinal section of the aerosol assembly in Fig. 3 with the mouthpiece attached to it;
Fig. 7 is a close-up view of the aerosol assembly in Fig. 6;
Fig. 8 is an example of an elastic sealing member; and
Fig. 9 shows a longitudinal section of another example of a medicament delivery device, not being covered by the subject-matter of the claims.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

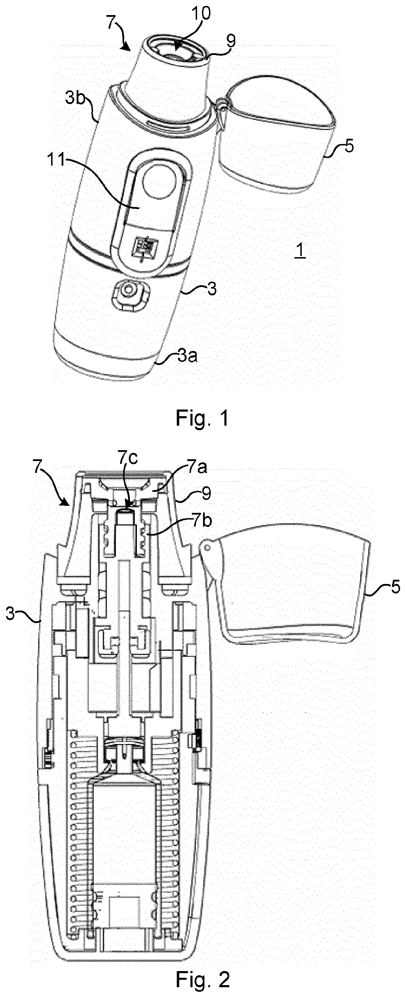

Fig. 1 depicts a perspective view of an example of a medicament delivery device 1. The medicament delivery device 1 is in this example an inhaler but could alternatively for example be an eye dispenser.

The medicament delivery device 1 comprises a housing 3. The housing 3 has a rear portion 3a and a front portion 3b. The medicament delivery device 3 also comprises a cap 5. In the present example, the cap 5 is hingedly connected to the front portion 3b of the housing 3.

The medicament delivery device 1 comprises an aerosol assembly 7. The aerosol assembly 7 forms a front portion of the medicament delivery device 1. The aerosol assembly 7 forms part of an aerosol interface for expulsion of medicament from the medicament delivery device 1 as an aerosol. The medicament delivery device 1 comprises a mouth piece 9. The mouth piece 9 is provided with a central through-opening 10. The central through-opening 10 is in fluid connection with interior of the medicament delivery device 1, specifically with the medicament container contained therein.

The cap 5 can be moved to a closed position in which it covers the aerosol assembly 7. This is normally the case when the medicament delivery device 1 is not in use. In Fig. 1, the cap 5 has been folded to the side to an open position by its hinged connection to expose the mouth piece 9. The cap 5 is set in the open position when the medicament delivery device 1 is to be used.

The mouth piece 9 may be placed against the mouth of the user of the medicament delivery device 1. The medicament delivery device 1 comprises an activation member 11 configured to trigger a medicament delivery. A user can activate the medicament delivery device 1 to achieve a medicament delivery by means of the activation member 11. A medicament is then expelled as an aerosol through the mouth piece 9.

Fig. 2 shows a longitudinal section of the medicament delivery device 1. The aerosol assembly 7 comprises a holding structure 7a attached to the mouth piece 9. The aerosol assembly 7 comprises a base member 7b configured to be connected to the holding structure 7a. Further, the aerosol assembly 7 comprises a nozzle assembly 7c, including a nozzle chip (not shown in Fig. 2) held by the holding structure 7a. The nozzle chip is configured to create an aerosol of medicament flowing through the nozzle chip when medicament administration is being performed. The nozzle chip is arranged such that the aerosol can exit the medicament delivery device 1 through the central through-opening of the mouth piece 9. The base member 7b extends into the housing 3 and is configured to fixate the holding structure 7a relative to the housing 3. The base member may for example form part of the "power pack" of the medicament delivery device 1, i.e. those components which provide the power for expelling the medicament, or it may be a component separate from the "power pack".

The general operation of the medicament delivery device 1 will not be discussed any further herein as the present disclosure is directed to the aerosol assembly 7, and the general operation of the medicament delivery device 1 would as such be apparent to the skilled person.

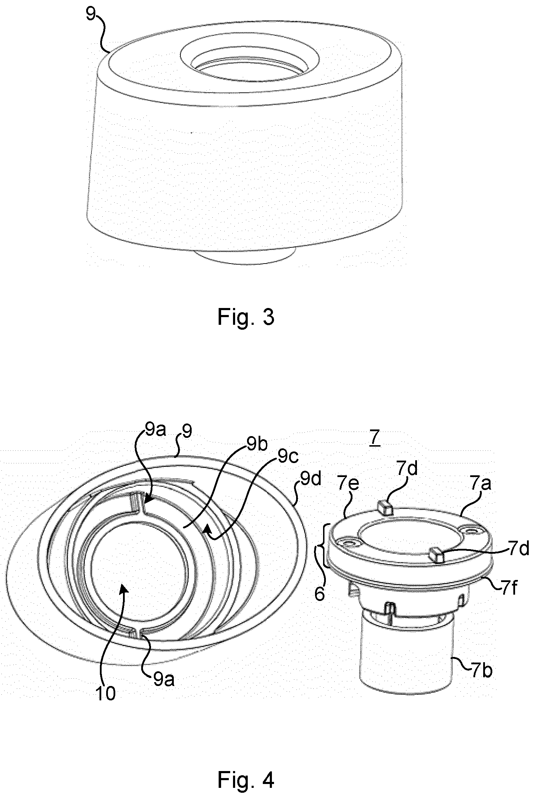

Fig. 3 depicts the aerosol assembly 7 and the mouth piece 9. Fig. 4 shows a bottom view of the mouth piece 9 to the left and the aerosol assembly 7to the right.

The mouth piece 9 is configured to receive the holding structure 7a. The holding structure 7a is arranged concentrically with the mouth piece 9. The mouth piece 9 is configured to be fixedly attached to the housing 3, to an internal component of the medicament delivery device 1 contained in the housing 3, or to the aerosol assembly 7, e.g. to the holding structure or to the base member. The mouth piece 9 is configured to be rotationally fixed relative to the housing 3. For example, the mouth piece 9 may be configured to engage with the housing 3 and/or an internal component of the medicament delivery device 1 by means of a snap-fit structure.

The mouth piece 9 is configured to prevent rotation of the holding structure 7a in a mounted state of the medicament delivery device 1. The mouth piece 9 has an inner surface 9b provided with first blocking structures 9a, which extend axially towards the holding structure 7a. The first blocking structures 9a may for example be axially extending protrusions or tabs configured to engage with the holding structure 7a. The holding structure 7a comprises second blocking structures 7d configured to cooperate with the first blocking structures 9a such that rotation of the holding structure 7a may be prevented. The second blocking structures 7d may be provided on a top surface 7e of the holding structure 7a, facing the inner surface 9b of the mouth piece 9. The second blocking structures 7d may be axially extending protrusions or tabs extending towards the inner surface 9b of the mouth piece 9. The second blocking structures 7d may for example be arranged at 180 degrees from each other on the top surface 7e of the holding structure 7a. The first blocking structures 9a may be arranged at corresponding locations on the inner surface 9b such that they engage with the second blocking structures 7d.

The first blocking structures 9a and the second blocking structures 7d are arranged relative to each other such that the mouth piece 9 can rotate some degrees relative to the holding structure 7a during assembly of the medicament delivery device 1 to attain their engaged position.

The holding structure 7a is provided with a sealing lip 7f. The sealing lip 7f extends along the entire perimeter of the holding structure 7a. The sealing lip 7f is configured to seal the holding structure 7 against the mouth piece 9. The mouth piece 9 comprises an inner wall 9c and an outer wall 9d. The inner wall 9c and the outer wall 9d are arranged concentrically. The outer wall 9d forms the outer surface of the mouth piece 9. The inner wall 9c extends around a top portion 6 of the holding structure 7a. According to the present example, the sealing lip 7f is configured to bear against the inner surface of the inner wall 9c to thereby seal the holding structure 7a against the mouth piece 9.

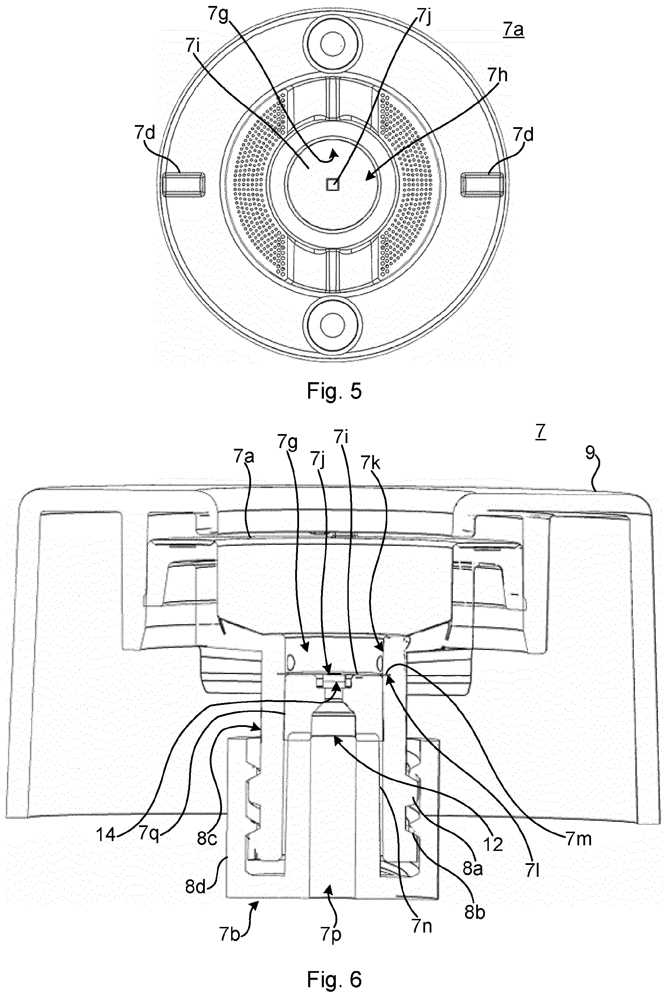

Fig. 5 shows a top view of the holding structure 7a. The holding structure 7a comprises a fluid channel 7g. The fluid channel 7g extends axially through the holding structure 7a. With the term "axial" is meant the central longitudinal axis of the medicament delivery device 1.

The aerosol assembly 7 includes the aforementioned nozzle chip assembly 7h. The nozzle chip assembly 7h comprises a carrier 7i and the nozzle chip 7j. The nozzle chip 7j is preferably a micro nozzle. The nozzle chip 7j is provided on the carrier 7i. The nozzle chip 7j is attached to the carrier 7i. The nozzle chip 7j may be bonded to the carrier 7i. For example, the nozzle chip 7j may be glued to the carrier 7i. The carrier 7i is a substrate. The carrier 7i is attached to the holding structure 7a. The carrier 7i is arranged fixed relative to the holding structure 7a. The carrier 7i is arranged in the fluid channel 7g such that medicament must flow through the nozzle chip 7j to form an aerosol. The nozzle chip 7j is hence arranged in the fluid channel 7g. The nozzle chip 7j may for example be centred in the fluid channel 7g, i.e. with respect to the central longitudinal axis of the medicament delivery device 1.

Fig. 6 depicts a longitudinal section of the aerosol assembly 7. An upper portion of the base member 7b is here shown in more detail. The base member 7b has a central channel 7p connected to the fluid channel 7g of the holding structure 7a.

The base member 7b a is configured to engage with the holding structure 7a. The base member 7b thereby holds the holding structure 7a in place relative to the housing 3. The fluid channel 7g extends through the holding structure 7a. One end of the fluid channel 7g opens into the central through-opening 10 of the mouth piece 9.

The fluid channel 7g has an inner surface 7k provided with an opening 7l. The opening 7l extends in the circumferential direction along the inner surface 7k. The opening 7l may be in the form of a slit. The opening 7l is configured to receive an outer boundary portion 7m of the carrier 7i. The carrier 7i is thereby fixed to the holding structure 7a inside the fluid channel 7g.

The base member 7b has an axially extending Luer cone 7n. The Luer cone 7n extends into the fluid channel 7g towards the carrier 7i and the nozzle chip 7j. The Luer cone 7n is configured to engage with or bear against the inner surface 7k of the fluid channel 7g. The Luer cone 7n has the central channel 7p. The central channel 7p extends axially in the Luer cone 7n. The central channel 7p opens into the fluid channel 7g towards the nozzle chip 7j. The Luer cone 7n does not extend all the way to the carrier 7i and the nozzle chip 7j.

The aerosol assembly 7 comprises an elastic sealing member 7q. The sealing member 7q is configured to seal the nozzle chip 7j towards the carrier 7i. The sealing member 7q may be rotationally symmetric. The sealing member 7q is made of a viscoelastic material. The viscoelastic material may be an elastomer. The sealing member 7q is arranged with a tight fit in the fluid channel 7g. The sealing member 7q is arranged axially between the Luer cone 7n and the carrier 7i. The sealing member 7q may bear against the Luer cone 7n and the carrier 7i.

The sealing member 7q is provided with a central through-opening 12 arranged coaxially with the central channel 7p of the Luer cone 7n and the fluid channel 7g of the holding structure 7a. In the present example, the central through-opening 12 has a varying diameter in the axial direction of the sealing member 7q. The diameter of the central through-opening 12 tapers in a direction towards the carrier 7i. The diameter of the central through-opening 12 is the same or essentially the same as the diameter of the central channel 7p as the central channel 7p transitions into the central through-opening 12. The diameter of the central through-opening 12 then tapers and obtains a first constant diameter. The diameter then widens in a direction towards the carrier 7i, for example in a discrete step to a second constant or essentially constant diameter forming a nozzle chip cavity 14. The nozzle chip 7j is arranged in the central through-opening 12. In the example in Fig. 6 the nozzle chip 7j is arranged in the central through-opening 12 where the central through-opening 12 has attained the second diameter, i.e. in the nozzle chip cavity 14.

The holding structure 7a is provided with first Luer threads 8a. The base member 7b is provided with second Luer threads 8b. The first Luer threads 8a are configured to engage with the second Luer threads 8b. The first Luer threads 8a, the second Luer threads 8b and the Luer cone 7n form a Luer lock connection between the holding structure 7a and the base member 7b.

The holding structure 7a has a hollow generally cylindrical end portion 8c. The cylindrical end portion 8c is provided with the first Luer threads 8a. In particular, the cylindrical end portion 8c has an external surface provided with the first Luer threads 8c. The Luer cone 7n is configured to be received into the fluid channel 7g in the cylindrical end portion 8c. The Luer cone 7n extends towards the carrier 7i. The base member has an outer wall 8d arranged radially outside of and concentrically with the Luer cone 7n. The outer wall 8d is provided with the second Luer threads 8b on its inner surface which faces the Luer cone 7n. A portion of the cylindrical end portion 8c provided with the first Luer threds 8a is arranged concentrically between the outer wall 8d and the Luer cone 7n. The first Luer threads 8a thereby engage with the second Luer threads 8b.

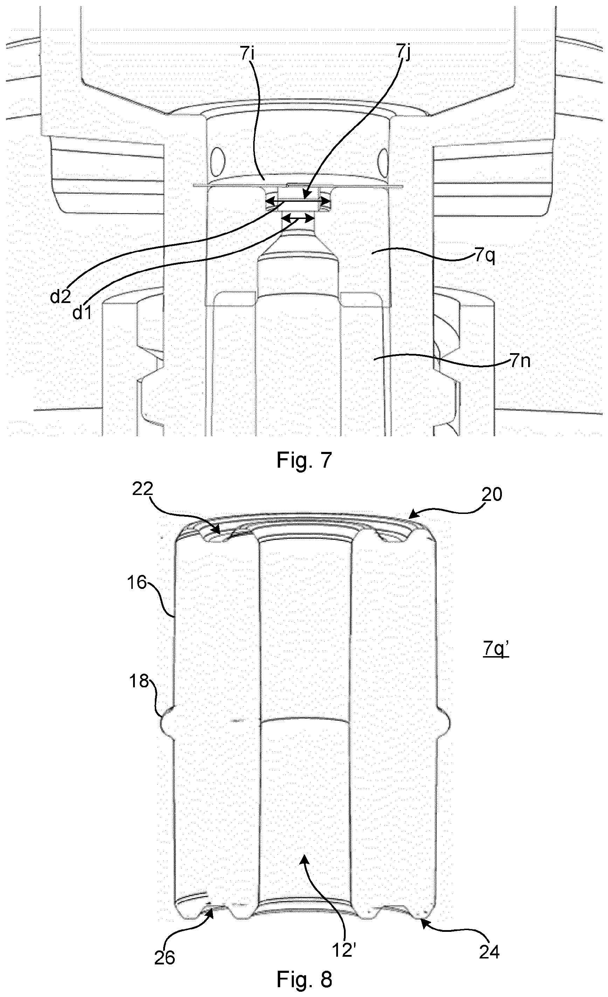

Fig. 7 shows a close-up view of the region around the nozzle chip 7j. It can here better be seen how the diameter of the central through-opening 12 varies in an axial direction towards the carrier 7i. In the depicted example, the first constant diameter of the central through-opening 12 is smaller than the corresponding dimensions of the nozzle chip 7j. All the liquid flowing through the central through-opening 12 towards the nozzle chip 7j will thereby pass through the nozzle chip 7j. The nozzle chip 7j may be sandwiched between the heel formed due to the step between the first diameter d1 and the second diameter d2 and the carrier 7i.

Fig. 8 shows another example of a sealing member 7q'. The sealing member 7q' has a central through-opening 12' that has a constant diameter along the entire length of the sealing member 7q'. The sealing member 7q' has an external surface 16 provided with a rib 18 extending in the circumferential direction of the sealing member 7q'. The rib 18 may extend along the entire circumference of the sealing member 7q'. The rib 18 may for example be arranged axially at or essentially at the mid-point between the two axial ends of the sealing member 7q'.

The sealing member 7q' has a first end surface 20 which bears against the carrier 7i. The first end surface 20 forms a first axial end of the sealing member 7q'. The first end surface 20 is provided with a first groove 22 which extends in the circumferential direction around the central through-opening 12'.

The sealing member 7q' has a second end surface 24 which bears against an end face of the Luer cone 7n. The second end surface 24 forms a second axial end of the sealing member 7q', opposite to the first axial end. The second end surface 24 is provided with a second groove 26 which extends in the circumferential direction around the central through-opening 12'.

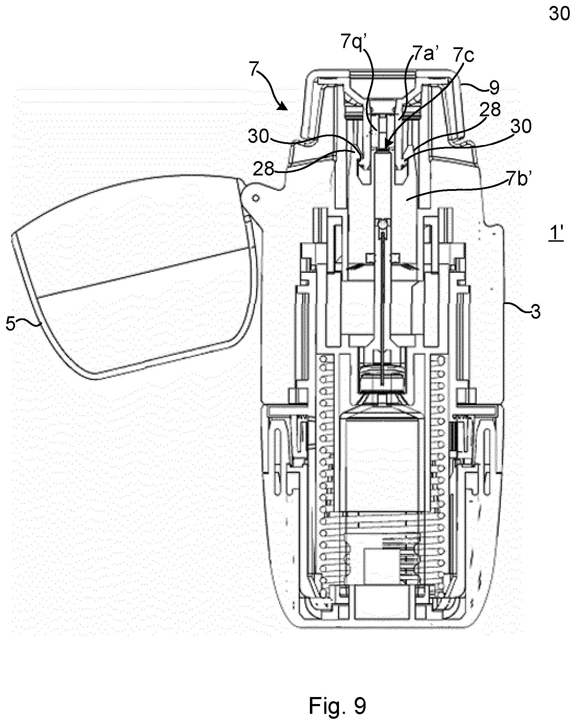

Fig. 9 shows another example of a medicament delivery device 1', not being covered by the subject-matter of the claims. In this example, the base member 7b' and the holding structure 7a' are configured to interlock with each other by means of a snap-fit mechanism.

The base member 7a' is provided with a plurality of gripping arms 28 and the holding structure 7a' is provided with radial protrusions 30. Each gripping arm 28 is configured to engage with a respective radial protrusion 30. The gripping arms 28 and the radial protrusions 30 form the snap-fit mechanism. The radial protrusions 30 extend radially from the outer surface of the cylindrical end portion of the holding structure 7a'. The base member 7b' has a cylindrical base member end portion configured to be received by the fluid channel of the holding structure 7a'. The sealing member 7q or 7q' is configured to be arranged between the carrier and the end face of the cylindrical base member end portion.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. An aerosol assembly (7) for a medicament delivery device (1), the aerosol assembly (7) comprising:
a nozzle chip assembly (7h) including a carrier (7i) and a nozzle chip (7j) provided on the carrier (7i),
a holding structure (7a; 7a') having a fluid channel (7g) extending through the holding structure (7a; 7a'),
wherein the nozzle chip assembly (7h) is configured to be mounted into the fluid channel (7g) such that fluid flowing through the fluid channel (7g) passes through the nozzle chip (7j), and
an elastic sealing member (7q; 7q') configured to be arranged with a tight fit in the fluid channel (7g), the sealing member (7q; 7q') having a central through-opening (12; 12') configured to receive the nozzle chip (7j) to seal the nozzle chip (7j) towards the carrier (7i).
wherein the fluid channel has (7g) an inner surface provided with a circumferentially extending opening(7l), wherein the carrier (7i) has an outer boundary portion (7m) mounted into the opening (7l) to hold the carrier (7i) in place;
wherein the aerosol assembly (7) comprises a base member (7b) having a Luer cone (7n) configured to extend into the fluid channel (7g) towards the nozzle chip assembly (7h), the Luer cone (7n) having a central channel (7p) extending through the base member (7b) and configured to extend coaxially with the fluid channel (7g), wherein the base member (7b) and the holding structure (7a) have Luer threads (8a, 8b) by which the base member (7b) and the holding structure (7a) are configured to be connected, wherein the central through-opening (12; 12') of the sealing member (7q; 7q') is configured to open towards the central channel (7p), and wherein the sealing member (7q; 7q') is configured to be arranged axially between the Luer cone (7n) and the carrier (7i).

2. The aerosol assembly (7) as claimed in claim 1, wherein the sealing member (7q; 7q') is configured to be in contact with the Luer cone (7n) and with the carrier (7i).

3. The aerosol assembly (7) as claimed in any of the preceding claims, wherein the sealing member (7q; 7q') comprises a viscoelastic material.

4. The aerosol assembly (7) as claimed in claim 3, wherein the viscoelastic material is an elastomer.

5. The aerosol assembly (7) as claimed in any of the preceding claims, wherein the sealing member (7q; 7q') is rotationally symmetric.

6. The aerosol assembly (7) as claimed in any of the preceding claims, wherein the central through-opening (12') has a constant inner diameter along its entire extension.

7. The aerosol assembly (7) as claimed in any of the preceding claims, wherein the sealing member (7q; 7q') is configured to be mounted in the fluid channel (12; 12') with a mounting compression of between 0.2 to 0.6 mm.

8. The aerosol assembly (7) as claimed in any of the preceding claims, wherein the sealing member (7q') has an external surface (16) provided with a rib (18) extending in a circumferential direction of the sealing member (7q').

9. The aerosol assembly (7) as claimed in any of the preceding claims, wherein the holding structure (7a) has a hollow generally cylindrical end portion (8c) in which the fluid channel (12; 12') extends axially, wherein the cylindrical end portion (8c) has an external surface provided with first Luer threads (8a), and wherein the base member (7b) has an outer wall (8d) arranged concentrically with the Luer cone (7n), wherein the cylindrical end portion (8c) is configured to be arranged concentrically between the Luer cone (7n) and the outer wall (8d), the outer wall (8d) having second Luer threads (8b) configured to engage with the first Luer threads (8a).

10. A medicament delivery device (1) comprising an aerosol assembly (7) as claimed in any of claims 1 - 9.

11. The medicament delivery device (1) as claimed in claim 10, wherein the medicament delivery device (1) is an inhaler.

## Patentansprüche

1. Aerosolanordnung (7) für eine Medikamentenabgabevorrichtung (1), die Aerosolanordnung (7) umfassend:
eine Düsenchipanordnung (7h), einschließlich eines Trägers (7i) und eines Düsenchips (7j), der auf dem Träger (7i) bereitgestellt ist,
eine Haltestruktur (7a; 7a'), die einen Fluidkanal (7g), der sich durch die Haltestruktur (7a; 7a') erstreckt, aufweist,
wobei die Düsenchipanordnung (7h) konfiguriert ist, um in den Fluidkanal (7g) montiert zu werden, sodass Fluid, das durch den Fluidkanal (7g) fließt, durch den Düsenchip (7j) hindurchgeht, und
ein elastisches Dichtungselement (7q; 7q'), das konfiguriert ist, um mit einer engen Passung in dem Fluidkanal (7g) eingerichtet zu sein, wobei das Dichtungselement (7q; 7q') eine zentrale Durchgangsöffnung (12; 12') aufweist, die konfiguriert ist, um den Düsenchip (7j) aufzunehmen, um den Düsenchip (7j) zu dem Träger (7i) hin abzudichten.
wobei der Fluidkanal (7g) eine Innenoberfläche aufweist, die mit einer sich umlaufend erstreckenden Öffnung (7l) versehen ist, wobei der Träger (7i) einen Außenbegrenzungsabschnitt (7m) aufweist, der in der Öffnung (7l) montiert ist, um den Träger (7i) an seinem Platz zu halten;
wobei die Aerosolanordnung (7) ein Basiselement (7b) umfasst, das einen Luer-Konus (7n) aufweist, der konfiguriert ist, um sich in den Fluidkanal (7g) zu der Düsenchipanordnung (7h) hin zu erstrecken, wobei der Luer-Konus (7n) einen zentralen Kanal (7p) aufweist, der sich durch das Basiselement (7b) erstreckt und konfiguriert ist, um sich koaxial zu dem Fluidkanal (7g) zu erstrecken, wobei das Basiselement (7b) und die Haltestruktur (7a) Luer-Gewinde (8a, 8b) aufweisen, mittels der das Basiselement (7b) und die Haltestruktur (7a) konfiguriert sind, um verbunden zu werden, wobei die zentrale Durchgangsöffnung (12; 12') des Dichtungselements (7q; 7q') konfiguriert ist, um sich zu dem zentralen Kanal (7p) hin zu öffnen, und wobei das Dichtungselement (7q; 7q') konfiguriert ist, um axial zwischen dem Luer-Konus (7n) und dem Träger (7i) eingerichtet zu sein.

2. Aerosolanordnung (7) nach Anspruch 1, wobei das Dichtungselement (7q; 7q') konfiguriert ist, um mit dem Luer-Konus (7n) und mit dem Träger (7i) in Kontakt zu sein.

3. Aerosolanordnung (7) nach einem der vorstehenden Ansprüche, wobei das Dichtungselement (7q; 7q') ein viskoelastisches Material umfasst.

4. Aerosolanordnung (7) nach Anspruch 3, wobei das viskoelastische Material ein Elastomer ist.

5. Aerosolanordnung (7) nach einem der vorstehenden Ansprüche, wobei das Dichtungselement (7q; 7q') rotationssymmetrisch ist.

6. Aerosolanordnung (7) nach einem der vorstehenden Ansprüche, wobei die zentrale Durchgangsöffnung (12') entlang ihrer gesamten Erstreckung einen konstanten Innendurchmesser aufweist.

7. Aerosolanordnung (7) nach einem der vorstehenden Ansprüche, wobei das Dichtungselement (7q; 7q') konfiguriert ist, um mit einer Montagekompression von zwischen 0,2 und 0,6 mm in dem Fluidkanal (12; 12') montiert zu werden.

8. Aerosolanordnung (7) nach einem der vorstehenden Ansprüche, wobei das Dichtungselement (7q') eine äußere Oberfläche (16) aufweist, die mit einer Rippe (18), die sich in einer umlaufenden Richtung des Dichtungselements (7q') erstreckt, versehen ist.

9. Aerosolanordnung (7) nach einem der vorstehenden Ansprüche, wobei die Haltestruktur (7a) einen hohlen, im Allgemeinen zylindrischen Endabschnitt (8c) aufweist, in dem sich der Fluidkanal (12; 12') axial erstreckt, wobei der zylindrische Endabschnitt (8c) eine äußere Oberfläche, die mit ersten Luer-Gewinden (8a) versehen ist, aufweist und wobei das Basiselement (7b) eine Außenwand (8d), die konzentrisch zu dem Luer-Konus (7n) eingerichtet ist, aufweist, wobei der zylindrische Endabschnitt (8c) konfiguriert ist, um konzentrisch zwischen dem Luer-Konus (7n) und der Außenwand (8d) eingerichtet zu sein, wobei die Außenwand (8d) zweite Luer-Gewinde (8b) aufweist, die konfiguriert sind, um mit den ersten Luer-Gewinden (8a) in Eingriff zu stehen.

10. Medikamentenabgabevorrichtung (1), umfassend eine Aerosolanordnung (7) nach einem der Ansprüche 1 bis 9.

11. Medikamentenabgabevorrichtung (1) nach Anspruch 10, wobei die Medikamentenabgabevorrichtung (1) ein Inhalator ist.

## Revendications

1. Ensemble aérosol (7) pour un dispositif d'administration de médicament (1), l'ensemble aérosol (7) comprenant :
un ensemble de puce de buse (7h) comportant un support (7i) et une puce de buse (7j) placée sur le support (7i),
une structure de maintien (7a ; 7a') ayant un canal de fluide (7g) s'étendant à travers la structure de maintien (7a ; 7a'),
dans lequel l'ensemble de puce de buse (7h) est conçu pour être monté dans le canal de fluide (7g) de telle sorte que le fluide circulant dans le canal de fluide (7g) passe à travers la puce de buse (7j), et
un élément d'étanchéité élastique (7q ; 7q') conçu pour être agencé avec un ajustement serré dans le canal de fluide (7g), l'élément d'étanchéité (7q ; 7q') ayant une ouverture traversante centrale (12 ; 12') conçue pour recevoir la puce de buse (7j) pour sceller la puce de buse (7j) vers le support (7i).
dans lequel le canal de fluide a (7g) une surface interne pourvue d'une ouverture (7l) s'étendant sur la circonférence, dans lequel le support (7i) a une partie de limite externe (7m) montée dans l'ouverture (7l) pour maintenir le support (7i) en place ;
dans lequel l'ensemble aérosol (7) comprend un élément de base (7b) ayant un cône Luer (7n) conçu pour s'étendre dans le canal de fluide (7g) vers l'ensemble de puce de buse (7h), le cône Luer (7n) ayant un canal central (7p) s'étendant à travers l'élément de base (7b) et conçu pour s'étendre coaxialement avec le canal de fluide (7g), dans lequel l'élément de base (7b) et la structure de maintien (7a) ont des filets Luer (8a, 8b) par lesquels l'élément de base (7b) et la structure de maintien (7a) sont conçus pour être reliés, dans lequel l'ouverture traversante centrale (12 ; 12') de l'élément d'étanchéité (7q ; 7q') est conçue pour s'ouvrir vers le canal central (7p), et dans lequel l'élément d'étanchéité (7q ; 7q') est conçu pour être agencé axialement entre le cône Luer (7n) et le support (7i).

2. Ensemble aérosol (7) selon la revendication 1, dans lequel l'élément d'étanchéité (7q ; 7q') est conçu pour être en contact avec le cône Luer (7n) et avec le support (7i).

3. Ensemble aérosol (7) selon l'une quelconque des revendications précédentes, dans lequel l'élément d'étanchéité (7q ; 7q') comprend un matériau viscoélastique.

4. Ensemble aérosol (7) selon la revendication 3, dans lequel le matériau viscoélastique est un élastomère.

5. Ensemble aérosol (7) selon l'une quelconque des revendications précédentes, dans lequel l'élément d'étanchéité (7q ; 7q') est symétrique de manière rotative.

6. Ensemble aérosol (7) selon l'une quelconque des revendications précédentes, dans lequel l'ouverture traversante centrale (12') a un diamètre interne constant sur toute son extension.

7. Ensemble aérosol (7) selon l'une quelconque des revendications précédentes, dans lequel l'élément d'étanchéité (7q ; 7q') est conçu pour être monté dans le canal de fluide (12 ; 12') avec une compression de montage entre 0,2 et 0,6 mm.

8. Ensemble aérosol (7) selon l'une quelconque des revendications précédentes, dans lequel l'élément d'étanchéité (7q') a une surface externe (16) pourvue d'une nervure (18) s'étendant dans une direction circonférentielle de l'élément d'étanchéité (7q').

9. Ensemble aérosol (7) selon l'une quelconque des revendications précédentes, dans lequel la structure de maintien (7a) a une partie d'extrémité (8c) creuse généralement cylindrique où le canal de fluide (12 ; 12') s'étend axialement, dans lequel la partie d'extrémité (8c) cylindrique a une surface externe pourvue de premiers filets Luer (8a), et dans lequel l'élément de base (7b) a une paroi externe (8d) agencée concentriquement avec le cône Luer (7n), dans lequel la partie d'extrémité cylindrique (8c) est conçue pour être agencée concentriquement entre le cône Luer (7n) et la paroi externe (8d), la paroi externe (8d) ayant des seconds filets Luer (8b) conçus pour venir en prise avec les premiers filets Luer (8a).

10. Dispositif d'administration de médicament (1) comprenant un aérosol (7) selon l'une quelconque des revendications 1 à 9.

11. Dispositif d'administration de médicament (1) selon la revendication 10, dans lequel le dispositif d'administration de médicament (1) est un inhalateur.
